# EUROPÄISCHE PATENTSCHRIFT

(11) **EP 2 477 528 B1**
(45) Veröffentlichungstag und Bekanntmachung des Hinweises auf die Patenterteilung: **29.06.2016**
(21) Anmeldenummer: 10750056.3
(22) Anmeldetag: 26.08.2010
(51) Int. Cl.: A61B 1/00, A61B 5/00

(54) **HANDSTÜCK-KAMERA**
HANDPIECE CAMERA
ENSEMBLE CAMÉRA-PIÈCE À MAIN

(30) Priorität: 14.09.2009 DE 102009041151
(43) Veröffentlichungstag der Anmeldung: 25.07.2012
(73) Patentinhaber: Dürr Dental AG, 74321 Bietigheim-Bissingen (DE)
(72) Erfinder: GEBHARDT, Herbert, 74936 Siegelsbach (DE); MAIER, Raimund, 71732 Tamm (DE); LAIS, Peter, 74391 Erligheim (DE); DÜRRSTEIN, Martin, 74321 Bietigheim-Bissingen (DE); THOMS, Michael, 91054 Erlangen (DE)
(74) Vertreter: Ostertag, Reinhard
(86) Internationale Anmeldenummer: PCT/EP2010/005237
(87) Internationale Veröffentlichungsnummer: WO 2011/029530

(56) Entgegenhaltungen:
- EP-A2- 1 238 624
- WO-A1-2005/110206
- DE-A1- 3 619 195
- DE-A1- 19 806 984
- DE-A1-102007 013 355
- JP-A- 10 229 971
- US-A1- 2005 003 323

## Beschreibung

Derartige Kameras mit einem elektronischen Bildwandler kommen z.B. zur Diagnose an unzugänglichen Stellen des menschlichen und tierischen Körpers zum Einsatz. Verbreitet finden sie auch in der Zahnmedizin eine Anwendung.

Aus der DE 10 2004 024 494 A1 ist eine Kamera bekannt, die ein Gehäuse umfasst, in dem eine Beleuchtungseinrichtung und optische Elemente angeordnet sind. Am Kopf des Gehäuses ist ein Eintrittsfenster vorgesehen, in das von einem Prüfkörper reflektiertes Beobachtungslicht einfällt. Optische Elemente im Kopf- und Griffabschnitt der Kamera richten das Beobachtungslicht auf einen Bildwandler. Der Bildwandler ist mit einer Kameraelektronik verbunden, die Betriebs- und Speissignale für die Kamera bereitstellt und über eine Auswerteschaltung eine Bildaufbereitung beispielsweise zur Ausgabe auf einem Bildschirm durchführt.

Nachteilig ist an den bekannten Kameras, dass die im Kameragehäuse angeordneten Bauteile schwer zugänglich und ein Austausch nicht möglich ist. Für verschiedene Anwendungsfälle ist daher jeweils eine spezielle Kamera erforderlich, wodurch hohe Investitionskosten entstehen. Ferner muss bei einer für Handstück-Kameras unabdingbaren Sterilisation das gesamte Gerät sterilisiert werden, wodurch dieses für geraume Zeit nicht verfügbar ist und insgesamt nur sterilisierbare Komponenten enthalten darf.

Aus der EP 1 238 624 A2 ist daher eine Intraoralkamera bekannt, bei der ein Handstück eine abnehmbare Linseneinheit trägt.

Ferner sind aus der US 2005/003323 A1 verschiedene Ausführungsbeispiele von Intraoralkameras bekannt, bei welchen sowohl einzelne Teile des Kamerakopfes als auch der gesamte Kamerakopf einschließlich Bildwandler austauschbar ausgestaltet sind.

Aufgabe der Erfindung ist es demgegenüber, eine Handstück-Kamera zur Verfügung zu stellen, die im Hinblick auf eine einfache Reinigung verbessert ist.

Diese Aufgabe ist durch eine Kamera mit den Merkmalen des Anspruchs 1 gelöst.

Die erfindungsgemäße Handstück-Kamera umfasst ein Gehäuse mit einem Kopfteil, das mit einem Griffteil lösbar verbunden ist. Das Kopfteil ist damit vom Griffteil abnehm- und austauschbar. Das im Mundraum eines Patienten verunreinigte Kopfteil ist getrennt vom Griffteil sterilisierbar, wodurch die Kamera in Verbindung mit einem anderen Kopfteil weiterhin verfügbar ist.

Durch eine Verrastung ist eine sichere Verbindung zwischen Kopf- und Griffteil gegeben. Gleichzeitig ist auch ein schneller Wechsel des Kopfteils möglich. Eine Abdichtung wie beispielsweise ein O-Ring verhindert dabei ein Eindringen von Schmutz oder Feuchtigkeit in die Kamera.

Vorteilhafte Weiterbildungen der Erfindung sind in den Unteransprüchen angegeben.

Die Weiterbildung der Kamera nach Anspruch 2 ermöglicht eine bedarfsgerechte Anordnung einer Beleuchtungseinrichtung am Griffteil. Somit sind beispielsweise für intraorale und extrorale Aufnahmen Kopfteile mit unterschiedlich hell strahlenden Beleuchtungseinrichtungen am Griffteil aufsteckbar. Durch Einsatz eines Kopfteiles mit entsprechender Beleuchtungseinrichtung ist die Kamera für unterschiedliche Aufnahmen und Diagnosen verwendbar.

Mit der Weiterbildung der Erfindung nach Anspruchs 3 wird erreicht, dass das Griffteil für verschieden Aufnahmearten wie beispielsweise Makro- oder Weitwinkelaufnahmen durch Aufstecken unterschiedlicher Kopfteile verwendbar ist.

Eine Kamera nach Anspruch 4 ist durch einen Aufsatz eines Kopfteiles mit einem Filter für Gewebeuntersuchungen beispielsweise mit UV-Licht einsetzbar, wobei der Filter vom Prüfgewebe ausgestrahltes Fluoreszenzlicht durchlässt und das angregende UV-Licht ausfiltert.

Die Weiterbildung der Erfindung, wonach ein Eintrittsfenster des Kopfteils als Filter ausgebildet ist, zeigt den Vorteil, dass das Kopfteil wenig Bauteile umfasst und daher kostengünstig darstellbar ist.

Eine Kamera nach Anspruch 5 ermöglicht eine Aufnahme eines Bildes in Querrichtung zur Griffachse.

Die Kamera nach Anspruch 6 ermöglicht eine Wärmeableitung aus dem Kopfteil. Eine leitende Verbindung leitet die Wärme, die beispielsweise durch Abstrahlung der Beleuchtungseinrichtung entsteht, zum Griffteil. Eine Konzentration der Wärme und eine Überhitzung von Bauteilen im Kopfteil ist damit vermieden. Die wärmeleitende Verbindung ist beispielsweise als Aluminiumbrücke ausgeführt. Bevorzugt ist die Beleuchtungseinrichtung in einem Aluminiumträger angeordnet, der über die Aluminiumbrücke oder anderweitig geeigneten Werkstoffen mit dem Griffteil verbunden ist.

Eine Kamera mit einer elektrisch betriebenen Beleuchtungseinrichtung weist den Vorteil auf, dass nach Bedarf Kopfteile mit unterschiedlichen Beleuchtungseinrichtungen einsetzbar sind, wobei lediglich eine geeignete Stromversorgung bereitzustellen ist.

Eine Kamera nach Anspruch 7 hat den Vorteil, dass beim Aufsetzen eines Kopfteiles auf das Griffteile die Beleuchtungseinrichtung durch eine entsprechende Anordnung der Kontakte gleichzeitig an die Stromquelle des Griffteils angeschlossen ist.

Die Weiterbildung der Erfindung gemäß Anspruch 8 ermöglicht es, elektrische Verbraucher und elektrische Signalquellen auf dem austauschbaren Kopfteil vorzusehen und mit Leitungen im Inneren des Griffteiles lösbar zu verbinden, wobei die Kontakte beim Aufschieben des Kopfteiles sukkzessive in Eingriff gebracht werden, so dass ein leichteres Aufschieben des Kopfteiles auf das Griffteil erhalten wird.

Dabei ist mit der Weiterbildung der Erfindung gemäß Anspruch 9 gewährleistet, dass über die Kontakte erst dann ein Strom fließt, wenn alle Kontakte und Gegenkontakte richtig einander gegenüberstehen.

Auch die Weiterbildung der Erfindung gemäß Anspruch 10 ist im Hinblick auf ein Schließen und Öffnen der Verbindung zwischen Kopfteil und Griffteil mit kleinen Kräften von Vorteil, wobei trotzdem eine sichere elektrische Verbindung zwischen den Kontakten und Gegenkontakten gegeben ist.

Mit der Weiterbildung der Erfindung gemäß Anspruch 11 wird automatisch eine automatische Verrastung des Kopfteiles auf den Griffteil über die Kontaktanordnung erhalten.

Dabei ist die Weiterbildung der Erfindung gemäß Anspruch 12 insoweit vorteilhaft, als die Blattfederenden einen linienhaften Kontakt zu den abgerundeten Endabschnitten der Kontakte aufweisen.

Eine Kamera, bei der die Beleuchtungseinrichtung mindestens eine LED umfasst, weist ein Kopfteil auf, das neben geringen Herstellungskosten einen kompakten Aufbau bei geringem Gewicht aufweist. Gleichzeitig ist die Wärmeabstrahlung von LED's gering.

Die Kamera nach Anspruch 13 nimmt selbständig eine für jedes Kopfteil entsprechende Einstellung eines optischen Elementes und/oder eines elektrischen Elementes vor, wobei solche elektrischen Elemente sowohl Aktuatoren als auch elektronische Steuerungen umfassen können. Beispielsweise wird die Position von optischen Linsen oder der Blendenwert über eine entsprechende Stelleinrichtung automatisch eingestellt. Eine Fehleinstellung durch den Benutzer ist damit ausgeschlossen.

Bei einer Kamera nach Anspruch 14 sind Informationen über das Kopfteil in einem Transponder gespeichert. Ein Auslesegerät im Griffteil ermittelt berührungslos die im Transponder gespeicherten Daten und übermittelt diese an eine Steuereinrichtung, die ein Verstellung von optischen Elementen und/oder elektrischen Elementen der Kamera vornimmt.

Bei einer Kamera, bei der die Einstellmittel elektrische Kontakte umfassen, sind am Griffteil und/ oder Kopfteil elektrische Kontakte vorgesehen, wobei bei einer Kombination des Griffteils mit unterschiedlichen Kopfteilen jeweils andere Kontakte schließen. Das Schließen eines Kontaktes löst eine Verstellung eines Kameraelementes aus. Der Einsatz von im Handel als Massenware erhältlichen Kontakten stellt eine kostengünstige Lösung der Erkennung des Kopfteiltyps dar.

Bei einer Kamera, bei der die Einstellmittel einen Magnet umfassen, ist eine berührungslose Erkennung von verschiedenen Kopfteilen möglich. Die Nutzung der Magnetkraft ermöglicht eine direkte mechanische Verstellung von optischen Elementen. Weiter ist auch die Position des Magneten über Schaltkontakte bestimmbar, die zur Ansteuerung elektrischer Element nutzbar sind. Diese Art der Verstellung ist wenig fehleranfällig und robust. Ein mit dem Magneten berührungslos zusammenwirkender Reedschalter arbeitet reibungsfrei und beeinflußt die Bewegung des Magneten im Griffteil nicht.

Bei einer Kamera, bei der ein Magnet mit einer vor dem Bildwandler angeordneten Blende zusammenwirkt, verstellt sich die Blende in Abhängigkeit der Ausbildung des aufgesetzten Kopfteiles. Beispielsweise schließt sich die Blende bei einem Kopfteil für Makroaufnahmen selbständig. Eine mechanische Kopplung des Magneten mit der Blende ermöglicht eine stromlose Verstellung. Alternativ ist eine von Schaltkontakten ausgewertete Position des Magneten zur Ansteuerung eines die Blende verstellenden Elektromotors nutzbar. Die Mittel können selbstverständlich auch eine Verstellung weiterer optischer Elemente vornehmen, beispielsweise eine axiale Verschiebung einer Linse.

Bei einer Kamera nach Anspruch 15 erfolgt in Abhängigkeit des verwendeten Kopfteiles eine selbständige Einstellung von Funktionen der Bildaufnahme und Auswertung, d.h. die Kameraelektronik stellt selbständig beispielsweise eine richtige Verstärkung, einen Weißabgleich etc. ein. Eine Fehlbedienung durch den Benutzer ist damit ausgeschlossen.

Ausführungsbeispiele der Erfindung sowie Darstellungen hilfreich zum Verständnis der Erfindung sind in den Zeichnungen dargestellt und werden in der nachfolgenden Beschreibung näher erläutert. In der Zeichnung zeigen:
- Fig. 1: ein schematisiertes Schnittbild einer dentalen oder medizinischen Handstück-Kamera mit einem Griffteil und einem Kopfteil;
- Fig. 2: eine Variante des Kopfteils aus Fig. 1;
- Fig. 3: eine weitere Variante des Kopfteils auf Fig. 1;
- Fig. 4: eine Kamera mit einem Verstellmagneten in Neutrallage;
- Fig. 5: eine Kamera mit dem Verstellmagneten in einer ersten ausgelenkten Lage;
- Fig. 6: eine Kamera mit dem Verstellmagneten in einer zweiten ausgelenkten Lage;
- Fig. 7: einen Längsschnitt durch ein Ausführungsbeispiel der Erfindung, bei welchem der Bildwandler beim objektivseitigen Ende der Kamera vorgesehen ist;

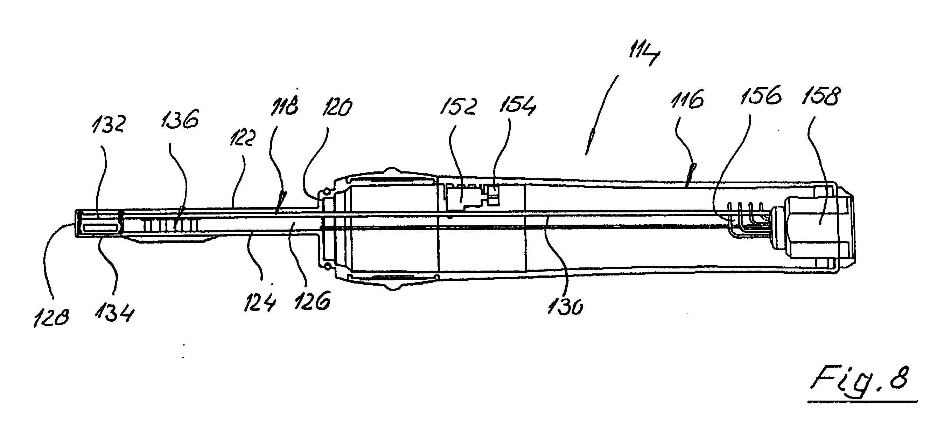

- Fig. 8: eine ähnliche Ansicht wie Figur 7, wobei jedoch das Kopfteil abgenommen ist;

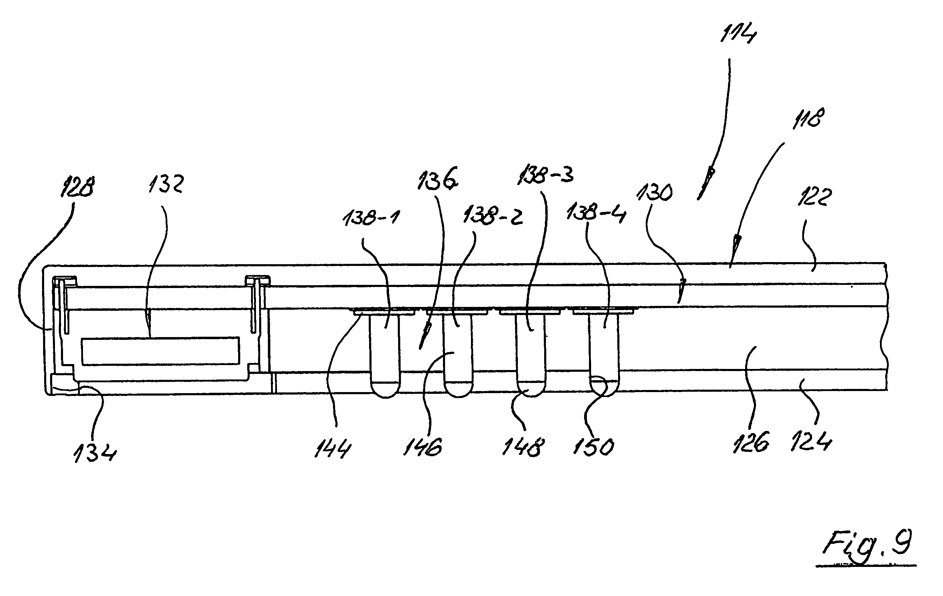

- Fig.9: eine vergrößerte Ansicht des freien Endabschnittes des Griffteiles in vergrößertem Maßstabe;
- Fig. 10: eine ähnliche Ansicht wie Figur 9, wobei jedoch ein Winkel-Kopfteil auf das Ende des Griffteiles aufgesetzt ist;
- Fig. 11: eine ähnliche Ansicht wie Figur 10, wobei jedoch anstelle des Winkel-Kopfteiles ein Geradsicht-Kopfteil auf das Griffteil aufgesetzt ist; und
- Fig. 12: ein abgewandeltes Geradsicht-Kopfteil in axialer Schnittansicht.

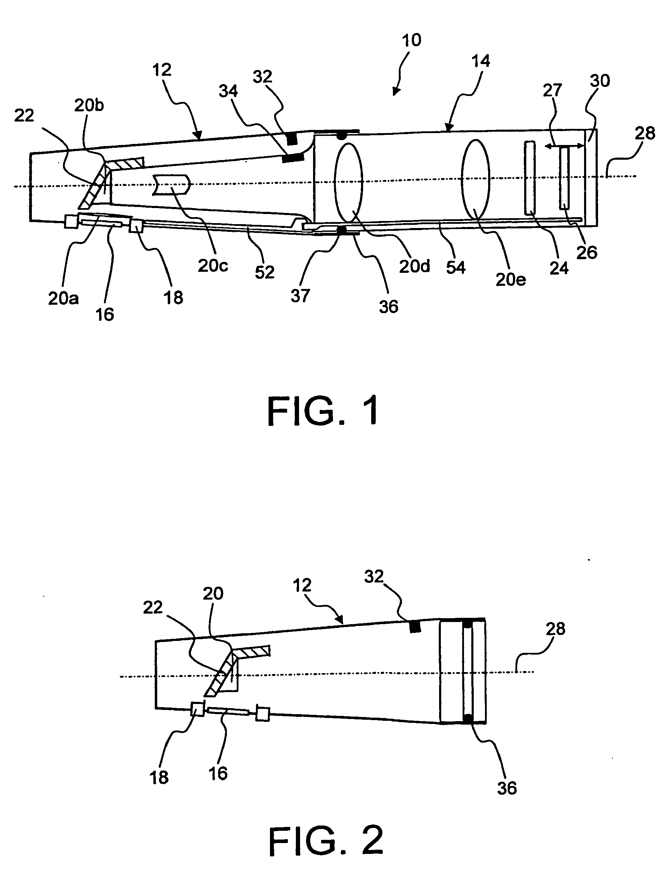

Die Fig.1 zeigt eine Handstück-Kamera, die beispielsweise im Mundbereich oder zur Betrachtung der Haut verwendbar ist.

Die Kamera weist ein Gehäuse 10 mit einem Kopfteil 12 und eieinem Griffteil 14 auf. In dem Kopfteil 12 ist ein Eintrittsfenster 16 angeordnet. In dem Kopfteil 12 ist eine ringförmige Lichtquelle 18 angeordnet, die Weißlicht erzeugt. Die Lichtquelle 18 umfasst mehrere um das Eintrittsfenster 16 verteilt angeordnete Leuchtdioden.

Eine erste Linse 20a ist beabstandet hinter dem Eintrittsfenster 16 im Kopfteil 12 angeordnet. Fest mit dem Kopfteil 12 verbunden ist ein Umlenkprisma 20b angeordnet. Zur Vermeidung von Beschädigungen ist auf dem Prisma 20b eine Schutzkappe 22 angeordnet.

Im Griffteil 14 sind weitere Linsen 20c, 20d, 20e und eine Blende 24 angeordnet, und hinter diesen Komponenten befindet sich ein Bildwandler 26.

Zur Einstellung der Bildschärfe ist eine schematisch bei 27 gezeigter Antrieb zum Verschieben des Bildwandlers 26 in Richtung der optischen Längsachse 28 des Griffteiles 14 vorgesehen.

Der Bildwandler 26 ist mit einer Kameraelektronik 30 verbunden, die eine Einstellung von Kamerakomponenten optischer und elektronischer Art als auch eine Darstellung und geeignete Bearbeitung eines aufgenommenen Bildes ermöglicht.

An dem Kopfteil 12 ist ein Transponder 32 angeordnet, der Informationen über den Aufbau des Kopfteiles 12 enthält. Sobald das Kopfteil 12 wie gezeichnet mit einem Griffteil 14 gepaart ist, liest ein im Griffteil 14 angeordnetes Lesegerät 34 diese Informationen aus und übermittelt diese an die Kameraelektronik 30 weiter. Aufgrund der übermittelten Informationen über den Aufbau des Kopfteiles 12 nimmt die Kameraelektronik 30 automatisch verschiedene Betriebseinstellung vor, beispielsweise eine Einstellung des Weißabgleichs, eine Einstellung der Spannung für die Beleuchtungseinheit 18, eine Einstellung der Blende 24, eine Einstellung einer Bildsignal-Verstärkung und/oder einer Fokussierung.

Das Kopfteil 12 weist auf der dem Griffteil 14 zugewandten Seite einen Rastwulst 36 auf, der in eine im Griffteil 14 vorgesehene Nut 38 unter Vorspannung eingeschnappt ist. Damit kann das Kopfteil 12 ohne Werkzeug unter leichter Kraftaufwendung vom Griffteil 14 abgenommen und durch ein anderes Kopfteil 12 ersetzt werden.

Bei der Untersuchung eines Prüfobjektes, beispielsweise eines Zahnes, wird das Eintrittsfenster 16 auf die zu untersuchende Stelle gerichtet. Von der Lichtquelle 18 abgestrahltes Licht trifft auf das Prüfobjekt auf. Das von der Lichtquelle abgestrahlte Weißlicht wird von dem Prüfkörper als Beobachtungslicht reflektiert. Das Beobachtunglicht tritt über das Eintrittsfenster 16 in die Kamera ein und wird durch das Umlenkprisma 20b über die Linsen 20c, 20d, 20e und die Blende 24 auf den Bildwandler 26 gerichtet.

Die Fig. 2 zeigt ein weiteres Kopfteil 12, das gegen das Kopfteil 12 in Fig. 1 austauschbar ist. Diese Variante des Kopfteiles 12 und auch die der weiteren Figuren sind der besseren Lesbarkeit halber mit dem gleichen Bezugszeichen bezeichnet, obgleich sich deren Aufbau unterscheidet.

Die gesamte aufwendige Optik und Elektronik des Griffteiles 14 der Fig. 1 ist mit dem Kopfteil 12 der Fig. 2 für weitere Untersuchungen nutzbar. Im Gegensatz zu dem Kopfteil 12 in Fig. 1 umfasst die Lichtquelle 18 nun UV-Leuchtdioden. Das UV-Licht regt etwa vom Prüfkörper getragene Bakterien optisch an, so dass diese als Beobachtungslicht Fluoreszenzlicht anderer Farbe abstrahlen. Anhand von Fluoreszenzlicht, das auf von Bakterien zurückgeht, sind gesunde und kranke Gewebebereiche unterscheidbar.

Das Eintrittsfenster 16 ist als Tiefpass-Kantenfilter ausgeführt, das das anregende UV-Licht vollständig absorbiert, so dass nur das Fluoreszenzlicht auf den Bildwandler 26 fällt.

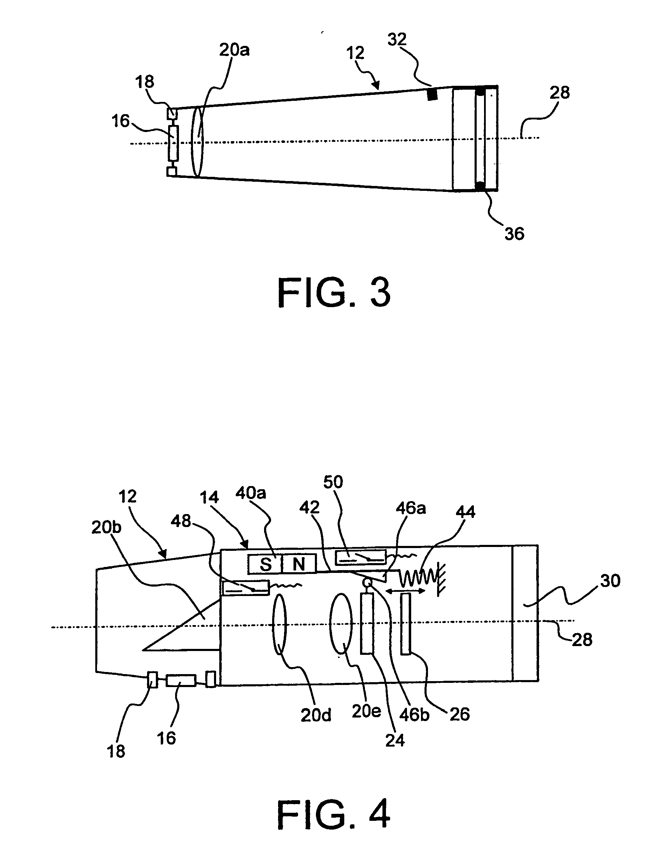

Fig. 3 zeigt eine weiteres Geradsicht-Kopfteil 12, das mit dem Griffteil 14 aus Fig.1 kombinierbar ist. Dieses Kopfteil 12 leitet das Beobachtungslicht ohne Umlenkung entlang der optischen Längsachse 28 in Richtung Bildwandler 26. Die Lichtquelle 18 umfasst besonders starke, beispielsweise für extraorale Aufnahmen geeignete Weißlichtleuchtdioden.

Die drei gezeigten Kopfteile 12 der Figuren 1 bis 3 stellen lediglich beispielhafte Ausführungsbeispiele dar, die mit dem Griffteil 14 aus Fig. 1 kombinierbar sind. Beispielsweise sind Kopfteile 12 mit weiteren Linsen für Weitwinkel- oder Makroaufnahmen sowie mit weiteren oder anderen Filtern darstellbar. Weiter können an den Kopfteilen 12 je nach Einsatzzweck unterschiedliche Lichtquellen 18 angeordnet sein, z.B. Infrarot- bzw. Laserlichtquellen, Lichtquellen unterschiedlicher oder einstellbarer Intensität oder gepulste Lichtquellen.

Ein weiteres Nutzungsfeld der Teilung der Kamera in Kopfteil und Griffteil erschließt sich durch Adapter, die einen Anschluss an ein Mikroskop oder ein Endoskop ermöglichen.

Die Fig.4 zeigt eine weitere Ausführungsform einer Kamera mit einem Kopfteil 12 und einem Griffteil 14. Die Anordnung der optischen Elemente 20b, 20d, 20e und 24 entspricht der in Fig. 1 beschriebenen Ausführung.

Das Kopfteil 12 umfasst eine Weißlichtdioden-Lichtquelle 18 und ein Eintrittsfenster 16.

Im Griffteil 14 ist ein in Richtung der optischen Längsachse 28 verschiebbarer Dauermagnet 40a angeordnet, wobei der Südpol in Richtung Kopfteil 12 zeigt. Der quaderförmige Magnet 40a ist über eine Stange 42 mit einer an der Gehäuseinnenseite abgestützten Feder 44 verbunden. Mit der Stange 42 gekoppelt ist eine Keil 46a, dessen Keilfläche in Abwandlung auch als Steuerkurve ausgebildet sein kann.

Auf der Oberfläche des Keiles 46a läuft eine durch Federkraft in der Zeichnung nach oben vorgespannte Rolle 46b, die einen nicht gezeichneten Verstellmechanismus der Blende 24 betätigt. Die Feder 44 hält den Magneten 40a in einer definierten Stellung, wodurch über das Keilelement 46a und die Rolle 46b ein definierter Blendenwert eingestellt ist.

Zwei Reedschalter 48, 50 sind beabstandet zum Magneten 40a angeordnet, so dass diese normalerweise geöffnet sind. Die Reedschalter 48, 50 sind über nicht dargestellte Kabel mit der Kameraelektronik 30 verbunden.

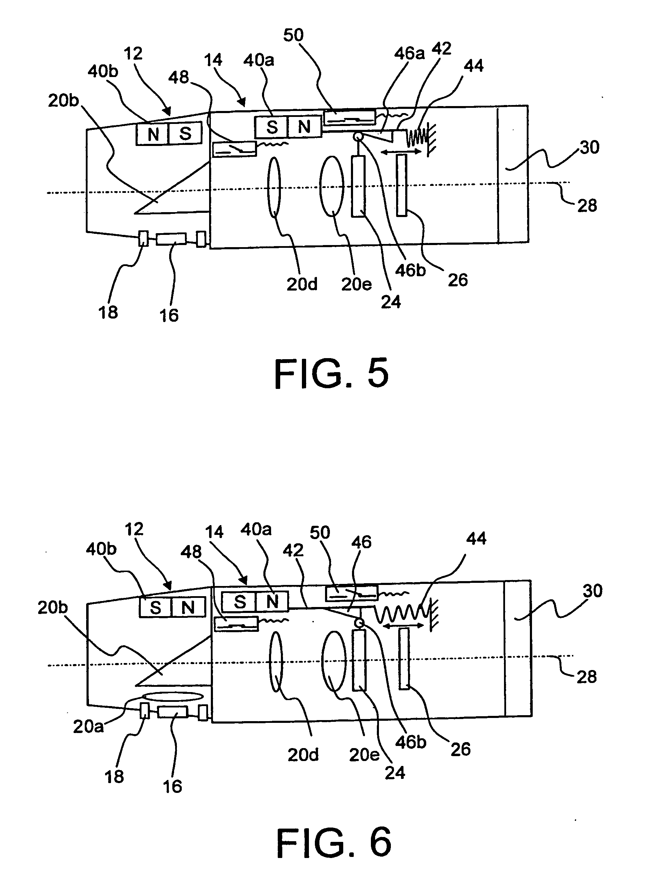

Die Fig. 5 zeigt das Griffteil 14 aus Fig. 4 in Kombination mit einem anderen Kopfteil 12.

Im Kopfteil 12 der Fig. 5 ist für Aufnahmen außerhalb der Mundhöhle eine stark leuchtende Leuchtdioden umfassende Lichtquelle 18 angeordnet. Im Kopfteil 12 ist im Gegensatz zu der Ausführung in Fig. 1 ein Magnet 40b angeordnet, dessen Südpol in Richtung Griffteil 14 gerichtet ist. Aufgrund der sich abstoßenden Pole des Magneten 40b und des Magneten 40a verschiebt sich letzterer beim Aufsetzten des Kopfteiles 12 auf das Griffteil 14 gegen die Kraft der Feder 44 in Richtung der Längsachse 28 vom Kopfteil 12 weg. Gleichzeitig verschiebt sich auch das Keilelement 46a in gleicher Richtung, womit die durch Federkraft vorgespannte Rolle 46b auf der Keilfläche nach oben läuft und damit die Blende 24 weiter öffnet.

Durch Annäherung des Magnete 40a verringert sich der Abstand zum Reedschalter 50, so dass dieser schließt. Die Kameraelektronik 30 erkennt den geschlossenen Kontakt 40a und stellt den Weißabgleich, Verstärkung etc. auf auf das Kopfteil 12 der Fig. 5 abgestimmte Werte ein.

In Fig. 6 ist wiederum das Griffteil 14 aus Fig. 5 gezeigt, das zusätzlich zum Aufbau des Kopfteils 12 aus Fig. 4 eine zum Eintrittsfenster 16 beabstandete optische Linse 20a aufweist, die beispielsweise für Makroaufnahmen geeignet ist.

Im Kopfteil 12 ist ferner ein Dauermagnet 40b angeordnet, dessen Nordpol dem Griffteil 14 zugewandt ist. Die Magnete 40b und 40a ziehen sich gegenseitig an, wodurch sich der Magnet 40a in Richtung Kopfteil 12 verlagert. Mit dem Magneten 40a verlagert sich wiederum der Keil 46a, die Rolle 46b rollt auf der Keilfläche in der Zeichnung nach unten und schließt die Blende 24 weiter.

Aufgrund der Vergrößerung des Abstands des Magneten 40a zum Reedkontakt 50 öffnet sich dieser. Der Abstand des Magneten 40a zum Reedkontakt 48 verringert sich, so dass dieser schließt. Die Signale der Reedkontakte 48, 50 wertet die Kameraelektronik 30 wie vorab beschrieben aus und nimmt entsprechende Einstellungen vor.

In einem alternativen nicht gezeigten Ausführungsbeispiel übernimmt die Kameraelektronik 30 aufgrund der Auswertung der Stellung der Reedkontakte 48, 50 die Verstellung der Blende 24 durch Ansteuerung eines elektrischen Antriebes.

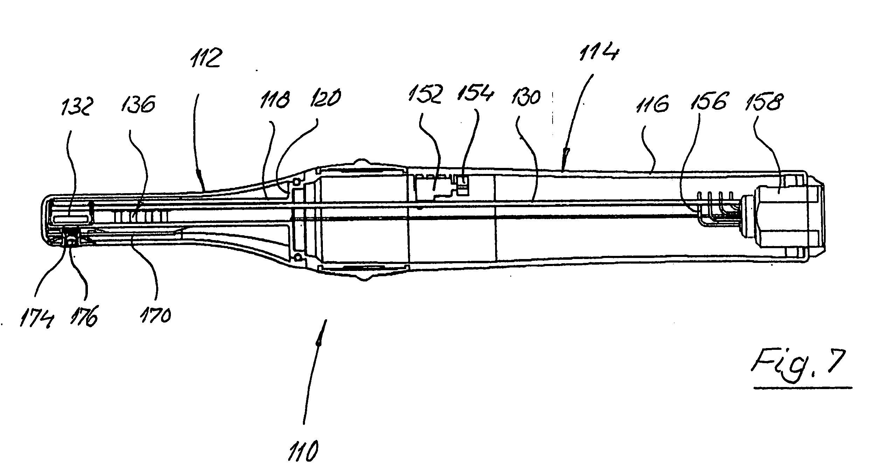

Das in den Figuren 7 ff. gezeigte Kamera-Handstück umfasst ein Gehäuse 110, zu dem ein lösbares Kopfteil 112 sowie ein Griffteil 114 gehören.

Das Griffteil 114 hat einen größeren Durchmesser aufweisenden Basisabschnitt 116 sowie einen von diesem getragenen schlanken langen Säulenabschnitt 118. Der Säulenabschnitt 118 ist auf einem schalenförmigen Befestigungsabschnitt 120 angeordnet und hat eine in Figur oben liegende erste achsparallele Hauptwand 122 sowie eine in Figur 7 unten liegende parallele Hauptwand 124. Deren Längskanten sind durch Seitenwände 126 miteinander verbunden, von denen nur eine in Figur 7 erkennbar ist.

Das in Figur 7 links liegende vordere Ende des Säulenabschnittes 118 ist durch eine Stirnwand 128 verschlossen.

Im Inneren des Säulenabschnittes 118 findet der Hauptwand 122 benachbart eine Hauptplatine 130 Aufnahme. Diese trägt bei ihrem freien Ende einen Kamera-Chip 132. Dessen Eingangsfenster ist durch ein Fenster 134 frei zugänglich, welches beim freien Ende der Hauptwand 124 vorgesehen ist.

Zusätzlich trägt die Hauptplatine 130 zwei Kontaktgruppen 136 mit Kontakten 138-1, 138-2, 138-3, 138-4, die paarweise senkrecht zur Zeichenebene der Figuren fluchtend hintereinanderliegen. Jeder der Kontakte 138 hat einen flanschförmigen Basisabschnitt 144, über den er mit einer Leiterbahn der Hauptplatine 130 mechanisch und elektrisch verbunden ist. Der Basisabschnitt 144 trägt jeweils einen Zapfenabschnitt 146, und die Zapfenabschnitte 146 haben jeweils einen kugelförmigen Endabschnitt 148. Die Enden der Zapfenabschnitte 146 und Endabschnitte 148 stehen in Öffnungen 150, die in der unteren Hauptwand 124 ausgebildet sind, derart, dass ein Teil der Endabschnitte 148 nach unten übersteht.

Die Hauptplatine 130 trägt ferner etwa bei ihrer Mitte einen Motor 152, der einen Unwuchtkörper 154 antreibt. Der Motor 152 wird erregt, wenn an einem am Gehäuse 110 angebrachten Taster eine Betätigung (z.B. für das Aufzeichnen eines Bildes) erfolgt, wodurch eine taktile Rückmeldung über den korrekten Empfang des Befehles erstellt wird.

Am in den Figuren rechts gelegenen Ende ist die Hauptplatine 130 über Leiter 156 mit einem Steckverbinderteil 158 verbunden, über welches ein Anschluss der Handstück-Kamera an eine externe Betriebs- und Auswerteeinheit folgt.

Das Kopfteil 112 hat grob gesprochen solche Form, dass es den Säulenabschnitt 118 unter geringem Spiel umgibt. Es hat eine obere Hauptwand 116, eine untere Hauptwand 162, Seitenwände 166, von denen wieder nur eine in der Zeichnung sichtbar ist, sowie eine Endwand 168. Diese Wände bilden zusammen, ähnlich wie die Wände des Säulenabschnittes 118 eine quaderförmige Struktur mit großzügig abgerundeten Längskanten und Querkanten.

Das Kopfteil 112 trägt in seinem Inneren eine Nebenplatine 170, die über zwei später noch genauer zu beschreibende Gegenkontaktgruppen 172 mit der Kontaktgruppe 136 zusammenarbeitet.

Die Gegenkontaktgruppen 172 umfassen Gegenkontakte 174-1, 174-2, 174-3 und 174-2, die paarweise fluchtend hintereinander liegen und deren Abstand dem Abstand der Kontakte 138 entspricht.

Bei ihrem freien, in der Zeichnung links gelegenen Ende ist die Nebenplatine 170 mit einer Öffnung 175 versehen, in welche ein Objektiv 176 fest eingesetzt ist.

Eine Objektivfassung ist außen abgestuft und nimmt dort einen hülsenförmigen Nabenabschnitt 178 einer kreisscheibenförmigen Fensterplatte 180 auf, die aus im Sichtbaren und ggf. im UV transparentem Kunststoffmaterial hergestellt ist. Die Fensterplatte 180 ist nicht eben, sondern stellt in verschiedenen Teilbereichen glatt Fortsetzungen der Außenfläche des Gehäuses 110 dar, so dass die Außenfläche des Gehäuses im Bereich der Fensterplatte 180 insgesamt glatt durchgehend ist.

Die Unterseite der Nebenplatine 170 trägt über der Fensterplatte 118 liegend einen Kranz von Leuchtdioden 182, die in Umfangsrichtung gleich verteilt sind und nach unten Weißlicht oder blaues oder ultraviolettes Licht abstrahlen. Es können auch zwei verschachtelte Sätze in Umfangsrichtung verteilter LEDs vorgesehen sein, von denen der eine Satz Weißlicht, der andere blaues Licht oder ultraviolettes Licht bereitstellt.

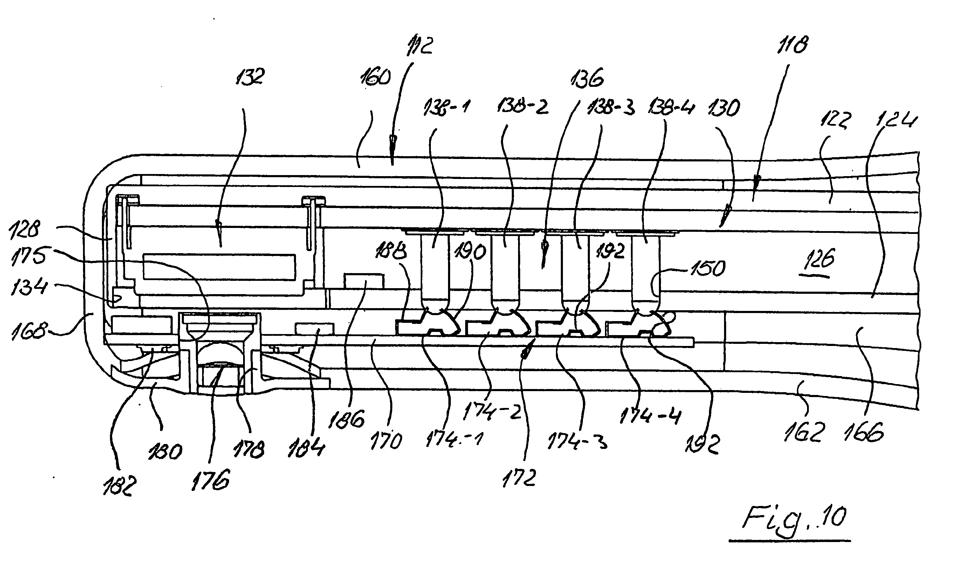

Auf der Oberseite der Nebenplatine 170 ist ein IC 184 erkennbar, der einen RFID oder einen anderen Transponder enthält, welcher Daten betreffend den Typ des Kopfteiles 112 und ggfs. noch weiterer Einzelheiten über das Kopfteil 112 enthält, die für das Auslesen des Kamera-Chips 132 und/oder für die Auswertung oder Bearbeitung des von diesem Chip aufgenommenen Bildes nützlich sind. Der IC 184 arbeitet mit einem entsprechenden Empfängerchip 186 zusammen, welcher auf der Hauptplatine 130 angeordnet ist, wie aus Figur 10 ersichtlich.

Die Kontaktfedern 172 sind aus einem streifenförmigen Blattfedermaterial hergestellt, welches gute elektrische Leitung aufweist. Die Kontaktfedern 170 haben grob gesprochen die Form eines Rechteckes, welches im oberen rechten Abschnitt aufgebrochen ist und dort zwei Federarme 188, 190 aufweist. Die Federarme 188, 190 sind an ihren freien Enden kreisbogenförmig passend zur Krümmung der Endabschnitte 148 gestaltet.

Die untere Seite des Rechteckes weit eine trapezförmige Eindrückung 192 auf, und der rechts der letzteren gelegene Bereich der unteren Rechteckseite ist konvex gewölbt, wie bei 194 gezeigt. Der Federarm 190 ist im unbelasteten Zustand ebenfalls konvex kreisbogenförmig gekrümmt, wie aus der Ausschnittvergrößerung von Figur 10 ersichtlich.

Die Kontakte 138 sowie die hierzu parallel hinter der Zeichenebene angeordneten Kontakte bilden die gehäusefeste Kontaktgruppe 136, während die Kontaktfedern 174 die bewegliche Gegenkontaktgruppe 172 bilden. Die Kontakte können sowohl zum Austausch von Information zwischen elektronischen Baueilen auf der Nebenplatine 170 und elektronischen Bauteile auf der Hauptplatine 130 dienen, als auch zur Ansteuerung von Aktoren oder anderen elektrischen Verbrauchern dienen, die auf der Nebenplatine angeordnet sind, als auch zur Spannungsversorgung auf der Nebenplatine angeordneter Lasten dienen.

Dabei sind die Kontakte 138-1, die beim Aufstecken des Kopfteiles 112 als letzte geschlossen werden, dazu bestimmt, die zum Betreiben der von der Nebenplatine 170 getragenen Bauteile notwendige Speisespannung zu übertragen. Das Aufstecken des Kopfteiles 112 erfolgt somit spannungsfrei, bis die Kontakte 138-1 mit den zugeordneten Gegenkontakten 174-1 in Eingriff kommen.

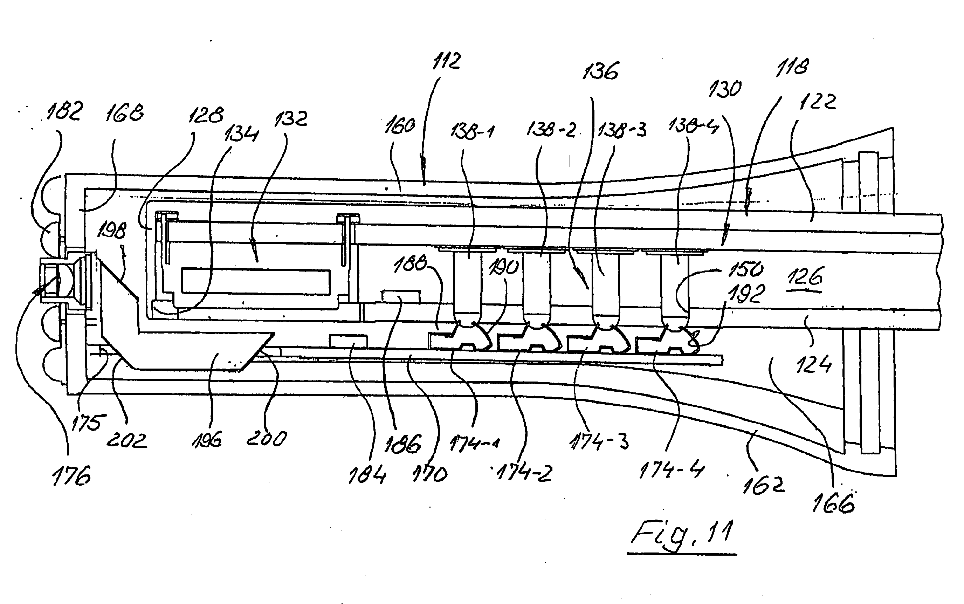

Beim Ausführungsbeispiel nach Figur 11 ist auf das Griffteil 114 ein Geradsicht-Kopfteil 112 aufgesetzt. Bei ihm fällt die Achse der Linsenanordnung 176 mit der Achse des Griffteiles 114 zusammen.

Das Zuführen des von der Beobachtungsstelle zurückgeführten Lichtes zum Kamera-Chip 132 erfolgt durch ein abgewinkeltes Prisma 196 aus transparentem optischen Material, welches bei den beiden Enden seiner Arme unter 45° angestellte Spiegelflächen 198, 200 aufweist und an der Stoßstelle der beiden Arme eine ebenfalls unter 45° angestellte Spiegelfläche 202 trägt. Diese Spiegel können dadurch realisiert sein, dass eine Verspiegelung auf entsprechend angestellten und geschliffenen Endflächen des Prismas 196 aufgebracht wird, z. B. durch Aufdampfen.

Ein weiterer Unterschied des Kopfteiles 112 nach Figur 11 zu demjenigen nach Figur 10 liegt darin, dass um das Objektiv herum ein Kranz von normalen Leuchtdioden 182 vorgesehen ist, die Weißlichtdioden, UV-Dioden oder Blaulicht-Dioden oder eine Mischung dieser Diodentypen sein können.

Im Übrigen sind in Figur 11 dasselbe Bezugszeichen verwendet wie in Figur 10. Die entsprechenden Komponenten brauchen nicht nochmals detalliert beschrieben zu werden.

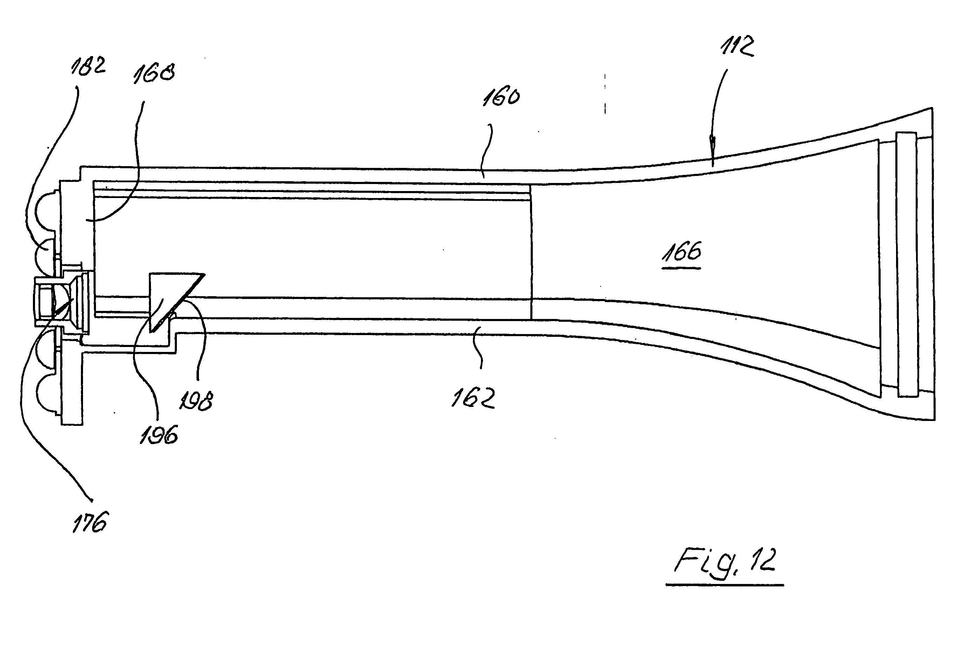

In Figur 12 ist ein nochmals abgewandeltes Kopfteil 112 dargestellt, welches ebenfalls ein Geradsicht-Kopfteil ist. Hier befindet sich die Achse der Linsenanordnung 176 etwas unterhalb der Achse des Griffteiles 114, was es erlaubt, das vom Untersuchtungsort zurückgeworfene Licht unter Verwendung eines einfachen Dreikant-Prismas 196 auf den Kamera-Chip 132 zu führen.

## Patentansprüche

1. Handstück-Kamera, die ein Gehäuse (10; 110), vom Gehäuse (10; 110) getragene optische Elemente (20a, 20b, 20c, 20d, 24; 176; 196) und einen im Gehäuse (10; 110) angeordnetten Bildwandler (26; 132) umfasst, wobei das Gehäuse (10; 110) ein Griffteil (14; 114) und ein lösbar mit diesem verbundenes Kopfteil (12; 112) umfasst,
**dadurch gekennzeichnet, dass**
a) das Griffteil (14; 114) einen Basisabschnitt (116) sowie einen schlankeren Säulenabschnitt (118) umfasst, in dessen Innerem eine Hauptplatine (130) aufgenommen ist, die bei ihrem freien Ende den Bildwandler (26; 132) trägt,
b) das lösbare Kopfteil (112) eine solche Form hat, dass es den Säulenabschnitt (118) umgibt, und dass
c) das Kopfteil (12; 112) mit dem Basisabschnitt (116) des Griffteils (14; 114) über eine Verrastung (36) verbunden ist.

2. Handstück-Kamera nach Anspruch 1 **dadurch gekennzeichnet, dass** eine Beleuchtungseinrichtung (18) von dem Kopfteil (12) getragen ist, die UV-Licht und/oder Weißlicht und/oder IR-Licht abstrahlt.

3. Handstück-Kamera nach Anspruch 1 oder 2 **dadurch gekennzeichnet, dass** mindestens eines (20a; 176; 196) der optischen Elemente (20a, 20b, 20c, 20d, 24; 176; 196) in dem Kopfteil (12; 112) angeordnet ist.

4. Handstück-Kamera nach Anspruch 3, **dadurch gekennzeichnet, dass** das mindestens eine optische Element ein Filter umfasst.

5. Handstück-Kamera nach Anspruch 3, **dadurch gekennzeichnet, dass** das mindestens eine optische Element eine Umlenkeinrichtung (20b) ist.

6. Handstück-Kamera nach einem der Ansprüche 1 bis 5, **dadurch gekennzeichnet, dass** zwischen dem Kopfteil (12) und dem Griffteil (14) eine thermisch leitende Verbindung vorgesehen ist.

7. Handstück-Kamera nach Anspruch 9, **dadurch gekennzeichnet, dass** eine elektrische Kontaktanordnung zwischen dem Kopfteil (12; 112) und dem Griffteil (14; 114) vorgesehen ist.

8. Handstück-Kamera nach Anspruch 7, **dadurch gekennzeichnet, dass** die elektrische Kontaktanordnung (136, 172) mindestens einen Satz, vorzugsweise zwei oder mehr Sätze in Steckrichtung des Kopfteiles (112) hintereinander liegender Kontakte (138, 174) aufweist.

9. Handstück-Kamera nach Anspruch 8, **dadurch gekennzeichnet, dass** zur Energieversorgung des Kopfteiles (112) dienende Kontakte (138-1, 174-1) die letzten beim Aufstecken des Kopfteiles (112) geschlossenen Kontakte sind.

10. Handstück-Kamera nach Anspruch 8 oder 9, **dadurch gekennzeichnet, dass** die Kontaktanordnung (136, 172) Kontakte (138) mit abgerundeten Endabschnitten (148) und mit diesen zusammenarbeitende Gegenkontakte (174) aufweist, die blattfederähnlich ausgebildet sind.

11. Handstück-Kamera nach Anspruch 10, **dadurch gekennzeichnet, dass** die Gegenkontakte (174) jeweils zwei Blattfederarme (188, 190) aufweisen, die von zwei Seiten her einen Kontakt (136 bis 142) zwischen sich aufnehmen.

12. Kamera-Handstück nach Anspruch 11, **dadurch gekennzeichnet, dass** die freien Enden der Blattfederarme (188, 190) freie Endkanten haben, die kreisbogenförmig gestaltet sind.

13. Handstück-Kamera nach einem der Ansprüche 1 bis 12, **dadurch gekennzeichnet, dass** Einstellmittel (40a, 40b, 48, 50; 34, 32) vorgesehen sind, die in Abhängigkeit von der Ausbildung des am Griffteil (14) angeordneten Kopfteils (12) auf ein optisches Element (20a, 20b, 20c, 20d, 24) und/oder ein elektrisches Element (30) wirken.

14. Handstück-Kamera nach Anspruch 13, **dadurch gekennzeichnet, dass** die Einstellmittel einen Transponder (32) und ein Transponder-Lesegerät (34) umfassen.

15. Handstück-Kamera nach einem der Ansprüche 13 bis 14, **dadurch gekennzeichnet, dass** die Einstellmittel mit einer mit dem Bildwandler (26) verbundenen Kameraelektronik (30) zusammenwirken, die in Abhängigkeit von der Ausbildung des Kopfteils (12) zu diesem gehörige Einstellungen zur Bildaufnahme und Auswertung vornimmt.

## Claims

1. Handpiece camera which includes a housing (10; 110), optical elements (20a, 20b, 20c, 20d, 24; 176; 196) carried by the housing (10; 110) and an image-converter (26; 132) arranged in the housing (10; 110), wherein the housing (10; 110) includes a handle member (14; 114) and a head member (12; 112) detachably connected to said handle member,
**characterised in that**
a) the handle member (14; 114) includes a base portion (116) and a more slender co-lumnar portion (118), wherein a main board (130) is received in the interior of said columnar portion, the main board supporting at its free end the image-converter (26; 132),
b) the detachable head member (112) has such a form that it surrounds the columnar portion (118), and **in that**
c) the head member (12; 112) is connected to the base portion (116) of the handle member (14; 114) via an interlocking detent (36).

2. Handpiece camera according to Claim 1, **characterised in that** an illuminating device (18) is carried by the head member (12), which emits UV light and/or white light and/or IR light.

3. Handpiece camera according to Claim 1 or 2, **characterised in that** at least one (20a; 176; 196) of the optical elements (20a, 20b, 20c, 20d, 24) is arranged in the head member (12; 112).

4. Handpiece camera according to Claim 3, **characterised in that** the at least one optical element includes a filter.

5. Handpiece camera according to Claim 3, **characterised in that** the at least one optical element is a redirecting device (20b).

6. Handpiece camera according to one of Claims 1 to 5, **characterised in that** a thermally conducting connection is provided between the head member (12) and the handle member (14).

7. Handpiece camera according to Claim 9, **characterised in that** an electrical contact arrangement is provided between the head member (12; 112) and the handle member (14; 114).

8. Handpiece camera according to Claim 7, **characterised in that** the electrical contact arrangement (136, 172) exhibits at least one set, preferentially two or more sets, of contacts (138, 174) situated one behind the other in the direction of insertion of the head member (112).

9. Handpiece camera according to Claim 8, **characterised in that** contacts (138-1, 174-1) serving for supplying power to the head member (112) are the contacts closed last when the head member (112) is being fitted.

10. Handpiece camera according to Claim 8 or 9, **characterised in that** the contact arrangement (136, 172) comprises contacts (138) with rounded end portions (148) and comprises mating contacts (174) co-operating with said end portions, which are similar in form to leaf springs.

11. Handpiece camera according to Claim 10, **characterised in that** the mating contacts (172) each comprise two leaf-spring arms (188, 190) which accept between themselves from two sides a contact (136 to 142).

12. Camera handpiece according to Claim 14, **characterised in that** the free ends of the leaf-spring arms (188, 190) have free end edges which are configured in the form of a circular arc.

13. Handpiece camera according to one of Claims 1 to 12, **characterised in that** setting means (40a, 40b, 48, 50; 34, 32) are provided which, depending on the design of the head member (12) arranged on the handle member (14), act on an optical element (20a, 20b, 20c, 20d, 24) and/or on an electrical element (30).

14. Handpiece camera according to Claim 13, **characterised in that** the setting means include a transponder (32) and a transponder reader (34).

15. Handpiece camera according to one of Claims 13 to 14, **characterised in that** the setting means interact with camera electronics (30) connected to the image-converter (26), which as a function of the design of the head member (12) perform settings pertaining to said head member for image-recording and evaluation

## Revendications

1. Caméra-pièce à main qui comprend un boîtier (10 ; 110), des éléments optiques (20a, 20b, 20c, 20d, 24 ; 176 ; 196) portés par le boîtier (10 ; 110) et un convertisseur d'images (26 ; 132) disposé dans le boîtier (10 ; 110), le boîtier (10 ; 110) comprenant une partie poignée (14 ; 114) et une partie tête (12 ; 112) reliée à celle-ci de manière détachable,
**caractérisée en ce que**
a) la partie poignée (14 ; 114) comprend une section de base (116) ainsi qu'une section colonnaire (118) plus mince, à l'intérieur de laquelle est logée une platine principale (130) qui porte le convertisseur d'images (26 ; 132) à son extrémité libre,
b) la partie tête (112) détachable a une forme telle qu'elle entoure la section colonnaire (118), et que
c) la partie tête (12 ; 112) est reliée à la section de base (116) de la partie poignée (14 ; 114) par un dispositif d'encliquetage (36).

2. Caméra-pièce à main selon la revendication 1, **caractérisée en ce qu'**un dispositif d'éclairage (18) est porté par la partie tête (12), lequel émet une lumière UV et/ou une lumière blanche et/ou une lumière IR.

3. Caméra-pièce à main selon la revendication 1 ou 2, **caractérisée en ce qu'**au moins un (20a ; 176 ; 196) des éléments optiques (20a, 20b, 20c, 20d, 24 ; 176 ; 196) est disposé dans la partie tête (12 ; 112).

4. Caméra-pièce à main selon la revendication 3, **caractérisée en ce que** ledit au moins un élément optique comprend un filtre.

5. Caméra-pièce à main selon la revendication 3, **caractérisée en ce que** ledit au moins un élément optique comprend un dispositif de déviation (20b).

6. Caméra-pièce à main selon l'une des revendications 1 à 5, **caractérisée en ce qu'**une liaison thermoconductrice est prévue entre la partie tête (12) et la partie poignée (14).

7. Caméra-pièce à main selon la revendication 9, **caractérisée en ce qu'**un agencement de contact électrique est prévu entre la partie tête (12 ; 112) et la partie poignée (14 ; 114).

8. Caméra-pièce à main selon la revendication 7, **caractérisée en ce que** l'agencement de contact électrique (136, 172) présente au moins un jeu, de préférence deux ou plusieurs jeux de contacts (138, 174) disposés les uns derrière les autres dans la direction d'enfichage de la partie tête (112).

9. Caméra-pièce à main selon la revendication 8, **caractérisée en ce que** des contacts (138-1, 174-1) servant à l'alimentation en énergie de la partie tête (112) sont les derniers à être fermés lors de l'enfichage de la partie tête (112).

10. Caméra-pièce à main selon la revendication 8 ou 9, **caractérisée en ce que** l'agencement de contact (136, 172) présente des contacts (138) ayant des sections terminales arrondies (148) et des contacts complémentaires (174) coopérant avec ceux-ci, qui sont réalisés en forme de ressort à lame.

11. Caméra-pièce à main selon la revendication 10, **caractérisée en ce que** les contacts complémentaires (174) présentent chaque fois deux bras de ressort à lame (188, 190) qui reçoivent entre eux un contact (136 à 142) depuis deux côtés.

12. Caméra-pièce à main selon la revendication 11, **caractérisée en ce que** les extrémités libres des bras de ressort à lame (188, 190) ont des bords d'extrémité libre en forme d'arc de cercle.

13. Caméra-pièce à main selon l'une des revendications 1 à 12, **caractérisée en ce que** des moyens de réglage (40a, 40b, 48, 50 ; 34, 32) sont prévus, qui agissent sur un élément optique (20a, 20b, 20c, 20d, 24) et/ou un élément électrique (30) en fonction de la configuration de la partie tête (12) disposée sur la partie poignée (14).

14. Caméra-pièce à main selon la revendication 13, **caractérisée en ce que** les moyens de réglage comprennent un transpondeur (32) et un lecteur de transpondeur (34).

15. Caméra-pièce à main selon l'une des revendications 13 à 14, **caractérisée en ce que** les moyens de réglage coopèrent avec une électronique de caméra (30) reliée au convertisseur d'images (26), laquelle, en fonction de la configuration de la partie tête (12), effectue les réglages correspondant à celle-ci pour la capture et l'évaluation d'images.
